# EUROPEAN PATENT SPECIFICATION

(11) **EP 1 827 104 B1**
(45) Date of publication and mention of the grant of the patent: **16.04.2014**
(21) Application number: 05855196.1
(22) Date of filing: 22.12.2005
(51) Int. Cl.: A01N 45/00, A61K 31/355, A61K 31/34, A61K 31/205, A61K 31/195, A61K 31/015, A61K 31/375, A23K 1/16, A23K 1/18

(54) **METHODS FOR PROMOTING ORAL HEALTH IN ANIMALS**
VERFAHREN ZUR FÖRDERUNG DER MUNDGESUNDHEIT BEI TIEREN
METHODES PERMETTANT D'AMELIORER LA SANTE BUCCO-DENTAIRE CHEZ LES ANIMAUX

(30) Priority: 22.12.2004 US 20449
(43) Date of publication of application: 05.09.2007
(73) Proprietor: Hill's Pet Nutrition, Inc., Topeka, KS 66603 (US)
(72) Inventor: SCHERL, Dale, Scott, Lawrence, KS 66049 (US); GROSS, Kathy, Lynn, Topeka, KS 66618 (US); LOGAN, Ellen, Irene, Wamego, KS 66547 (US)
(74) Representative: Daniels, Jeffrey Nicholas
(86) International application number: PCT/US2005/046596
(87) International publication number: WO 2006/069241

(56) References cited:
- EP-A1- 0 380 367
- WO-A1-2004/012522
- WO-A2-2006/071919
- DE-A1- 10 354 940
- US-A- 5 032 384
- US-A- 5 376 374
- US-A- 6 156 355
- US-A1- 2002 054 857
- US-B1- 6 814 958
- US-B2- 6 503 483
- CLARKE DAVID E: "CLINICAL AND MICROBIOLOGICAL EFFECTS OF ORAL ZINC ASCORBATE GEL IN CATS", JOURNAL OF VETERINARY DENTISTRY, AMERICAN VETERINARY DENTAL SOCIETY, LOCKPORT, US, vol. 18, no. 4, 1 December 2001 (2001-12-01), pages 177-183, XP008070576, ISSN: 0898-7564
- CLARKE D.E.: 'Clinical and Microbiological Effects of Oral Zinc Ascorbate Gel in Cats' J. VET. DENT. vol. 18, no. 4, 2001, pages 177 - 183, XP008070576

## Description

### BACKGROUND OF THE INVENTION

### Field of the Invention

The present invention relates generally to compositions for use in promoting oral health in animals and particularly to the use of antioxidants for promoting oral health in animals.

### Description of the Related Art

Animals, including companion animals such as cats and dogs, require oral care. Poor oral health can cause pain and serious dental problems throughout life, as well as possibly lead to more serious illnesses, such as, e.g., heart and kidney disease. According to the American Veterinary Dental Society and leading veterinary dental specialists, 70% of cats and 80% of dogs have some form of gum disease by age 3. Unhealthy gums, including gingival inflammation or gingivitis, are considered to be a common oral health issue affecting companion animals.

Methods for promoting oral health are known. U.S. Patent No. 6,503,483 describes compositions and methods for treatment of gum disease in humans and animals by topical administration of an orally absorbable dental formulation comprising vitamin C. U.S. Patent No. 5,376,374 proposes alleviation of gum disease by administration of an oral rinse composition and dietary supplementation with minerals and vitamins including vitamins C and E. U.S. Patent No. 5,032,384 describes compositions and methods for treatment of periodontal disease using a combination of an arylpropionic nonsteroidal anti-inflammatory drug (NSAID) and an antioxidant. U.S. Patent No. 4,272,512 proposes topical use of oral compositions and methods for inhibiting symptoms of gingivitis using tranexamic acid and folic acid. Battino et al. (1999) Crit. Rev. Oral Biol. Med. 10(4), 458, review possible therapeutic effects of antioxidants in treating or preventing inflammatory periodontal disease. Clarke (2001) J. Vet. Dent. 19(4), 177, proposes topical use of zinc ascorbate gel as an oral antiseptic to improve feline oral health.

Despite the availability of oral care products for animals, providing proper oral care to an animal such as companion animals remains a challenge due to, among other things, inconvenience, inadequateness, difficulty, and expense. For example, routine veterinary dental examinations and cleaning can be expensive. Regular brushing by the animal caregiver, though beneficial, can be an inconvenient chore that is difficult to perform regularly. Conventional hard, crunchy dry foods, chew toys, and the like, fail to completely remove plaque and tartar at the gumline and are inadequate to promote periodontal health. There remains, therefore, a need for convenient and effective methods of promoting oral health in animals, particularly companion animals.

WO2004/012522 discloses compositions comprising epigallocatechin gallate (EGCG) and lactoferrin, optionally including also vitamin E, vitamin C and a carotenoid; or epigallocatechin gallate, vitamin E, and a carotenoid; and their use in pet feed for preventing or treating plaque, gingivitis, periodontal disease and oral malodour (halitosis), and for enhancing the antioxidative capacity in the whole organism.

EP0380367 discloses compositions comprising an antioxidant and an arylpropionic, nonsteroidal anti-inflammatory drug.

US2002/0654857 A1 discloses a dental formulation comprising from about 0 to 25 weight percent vitamin C and co-enzyme Q-10.

DE10354940 discloses an oral hygiene product comprising melatonin, and optionally further comprising vitamin C and vitamin E.

US 6,814,958 B1 discloses food compositions, oral compositions and pharmaceutical compositions for preventing or treating periodontosis, the compositions comprising (A) one or more extracts containing natural polyphenol; and (B) one or more members selected from the group consisting of vitamin C, vitamin E, vitamin A and beta-carotene; and methods for preventing or treating periodontosis using the above compositions.

US 5,376,374 discloses a composition which consists essentially of cayenne pepper, calendula, echinacea, goldenseal, propolis, vinegar, and water.

W02006/071919 discloses a kit suitable for administering antioxidants to an animal.

### SUMMARY OF THE INVENTION

The invention provides a composition for use in promoting oral health in an animal, which composition comprises at least one antioxidant, wherein the at least one antioxidant comprises vitamin C in an amount of from 350 to 500 ppm and vitamin E in an amount of from 1000 to 1500 ppm, and wherein the use comprises causing the animal to ingest the composition.

The present invention is defined by the claims. Subject matter outside the scope of the claims is not in accordance with the present invention and is provided for information only.

### DETAILED DESCRIPTION OF THE INVENTION

### Definitions

The term "animal" means any animal susceptible to or suffering from poor oral health or in need of methods for promoting oral health.

The term "oral health" herein refers to any oral condition involving the teeth and/or gums.

The expression "promoting oral health" means mitigation, prevention, or treatment of oral conditions associated with the gums and/or tissues/structures surrounding and supporting the teeth. Non-limiting examples of oral conditions herein include gingivitis (inflammation of the gums (gingival tissue)) and periodontitis (inflammation and/or infection present both in the gingiva and in the connective tissue that supports the teeth).

The term "single package" means that the components of a kit are physically associated in or with one or more containers and considered a unit for manufacture, distribution, sale, or use. Containers include, but are not limited to, bags, boxes, bottles, shrink wrap packages, stapled or otherwise affixed components, or combinations thereof. A single package may be containers of individual antioxidants physically associated such that they are considered a unit for manufacture, distribution, sale, or use.

The term "virtual package" means that the components of a kit are associated by directions on one or more physical or virtual kit components instructing the user how to obtain the other components, e.g., in a bag containing one component and directions instructing the user to go to a website, contact a recorded message, view a visual message, or contact a caregiver or instructor to obtain instructions on how to use the kit.

This invention is not limited to the particular methodology, protocols, and reagents described herein because they may vary. Further, the terminology used herein is for the purpose of describing particular embodiments only and is not intended to limit the scope of the present invention. As used herein and in the appended claims, the singular forms "a," "an," and "the" include plural reference unless the context clearly dictates otherwise. Similarly, the words "comprise", "comprises", and "comprising" are to be interpreted inclusively rather than exclusively.

Unless defined otherwise, all technical and scientific terms and any acronyms used herein have the same meanings as commonly understood by one of ordinary skill in the art in the field of the invention. Although any methods and materials similar or equivalent to those described herein can be used in the practice of the present invention, the preferred methods, devices, and materials are described herein.

All patents, patent applications, and publications mentioned herein are incorporated herein by reference to the extent allowed by law for the purpose of describing and disclosing the compounds, processes, techniques, procedures, technology, articles, and other compositions and methods disclosed therein that might be used with the present invention. However, nothing herein is to be construed as an admission that the invention is not entitled to antedate such disclosure by virtue of prior invention.

The invention provides compositions for promoting oral health in animals. Specifically, the invention is directed to a composition for use in promoting oral health in an animal, which composition comprises at least one antioxidant, wherein the at least one antioxidant comprises vitamin C in an amount of from 350 to 500 ppm and vitamin E in an amount of from 1000 to 1500 ppm, and wherein the use comprises causing the animal to ingest the composition. Without being held to a particular theory, it is believed that free radicals, as well as oxidative processes, are involved in the processes of inflammation. For example, cyclooxygenase enzymes provide an oxygenation step in conversion of arachidonic acid to the pro-inflammatory molecule prostaglandin E2. Reactive oxygen species, e.g., nitric oxide, are contributors to inflammatory processes. Antioxidants are believed to inhibit the inflammatory processes that cause, e.g., gingival inflammation, gingivitis, or periodontal disease.

Antioxidants useful in the present invention are any material that either directly quenches a free radical or indirectly causes a free radical to become quenched. Skilled artisans know that a variety of materials have free radical quenching or absorbing capacity. For example, the following are raw ingredients that are high in oxygen radical absorbing capacity ("ORAC") content: spinach, spinach pomace, tomato pomace, citrus pulp, grape pomace, carrot, carrot granules, broccoli, green tea, ginkgo biloba, corn gluten meal, algae, curcumin, astaxanthin, beta-carotene, glutathione, green tea, lutein, lycopene, N-acetylcysteine, polyphenols, soy isoflavones, S-adenosylmethionine, sulfur-containing amino acids, taurine, tocotrienols, folate, vitamin A, vitamin C and vitamin E.

The composition must comprise at least one antioxidant comprising vitamin C in an amount of from 350 to 500 ppm and vitamin E in an amount of from 1000 to 1500 ppm and may also comprise, in various embodiments, for example, vitamin A, lipoic acid, astaxanthin, beta-carotene, L-carnitine, coenzyme Q10, glutathione, lycopene, lutein, N-acetylcysteine, soy isoflavones, S-adenosylmethionine, taurine, tocotrienols, spinach, tomato, citrus fruit, grape, carrot, broccoli, green tea, ginkgo biloba, corn gluten meal, rice bran, algae, curcumin, marine oil, fruits, vegetables, yeast, carotenoids, flavonoids, polyphenols, or mixtures thereof.

Examples of an antioxidant food, a food product, or a component thereof, raw or otherwise, include spinach (for example, spinach pomace), tomato (for example, tomato pomace), citrus fruit (for example, citrus pulp), grape (for example, grape pomace), carrot (for example, carrot granules), broccoli, green tea, ginkgo biloba, corn gluten meal, rice bran, algae, curcumin, marine oil, or yeast (for example, selenium yeast), or mixtures thereof.

Lipoic acid can be present as alpha-lipoic acid or a lipoate salt or ester, or, for example, an isomer of lipoic acid as described in U.S. Patent No. 5,621,117. As used herein, "alpha-lipoic acid" is synonymous with "lipoic acid" Lipoic acid can be provided in various forms, for example, racemic mixture, salt(s), ester(s), and/or amide(s). Lipoic acid, if present in the composition, can be in an amount of at least about 100 ppm, at least about 50 ppm, or at least about 25 ppm, up to about 600 ppm or up to an amount which is not toxic to the animal.

L-carnitine can be present as L-carnitine or in a derivative form, for example, a salt (for example, hydrochloride), an ester (for example, fumarate ester or succinate ester), or as acetylated L-carnitine. L-carnitine, if present in the composition, can be in an amount of at least about 500 ppm, at least about 200 ppm, at least about 100 ppm, or at least about 50 ppm. A non-toxic maximum quantity can be employed, for example, less than about 5,000 ppm.

Carotenoids can be present in the composition, including retinol (vitamin A), retinal, retinoic acid, α-carotene, β-carotene, γ-carotene, δ-carotene, lutein, lycopene, lycophyll, lycoxanthin, rhodoxanthin, astaxanthin and cryptoxanthin. Vitamin A can be present as vitamin A or derivatives such as C₂₋₂₀ fatty acid esters of vitamin A and the like. Illustratively, β-carotene, if present in the composition, can be in an amount of about 1 to about 15 ppm.

Other antioxidants include about 0.1 to about 5 ppm selenium; at least about 1 ppm of lutein up to about 100 ppm or up to an amount that is not toxic to the animal; at least about 25 ppm of coenzyme Q10 up to about 2000 ppm or up to an amount that is not toxic to the animal; at least about 50 ppm of S-adenosylmethionine up to about 2000 ppm or up to an amount that is not toxic to the animal; at least about 500 ppm of taurine up to about 5000 ppm or up to an amount that is not toxic to the animal; at least about 25 ppm of soy isoflavone(s) up to about 5000 ppm or up to an amount that is not toxic to the animal; at least about 50 ppm of N-acetylcysteine up to about 1000 ppm or up to an amount that is not toxic to the animal; at least about 50 ppm of glutathione up to about 1000 ppm or up to an amount that is not toxic to the animal; and at least 50 ppm of ginkgo biloba extract up to about 1000 ppm or up to an amount that is not toxic to the animal.

In the compositions for use, the use comprises causing the animal to ingest a composition comprising an oral health promoting amount of vitamin C and vitamin E in combination.

Vitamin E can be provided in any suitable form, for example as a tocopherol, a mixture of tocopherols, and/or various derivatives thereof, such as an ester derivative, for example, acetate, succinate, or palmitate ester of vitamin E. Vitamin E, as used herein, includes forms and derivatives that provide vitamin E-like activity after ingestion by the animal. Vitamin E can be in alpha, beta, gamma, or delta configurations. Furthermore, vitamin E can be in either its d-stereoisomer configuration or as a racemic mixture. Therefore, except where the context demands otherwise, the term "vitamin E" is used generically herein to encompass any tocopherol or tocotriene compound, including any enantiomer or racemate thereof, and any mixture of such compounds, having vitamin E activity.

The vitamin E content of the compositions of the invention is from 1000 to 1500 ppm.

Vitamin C can be provided to the animal as ascorbic acid, for example L-ascorbic acid, or various derivatives thereof, for example, calcium phosphate salt of ascorbic acid, cholesteryl salt of ascorbic acid, or ascorbate-2-monophosphate. Salts of vitamin C include, for example, sodium salt, calcium salt, zinc salt, and ferrous salt. Esters include, for example, stearate, palmitate, and like derivatives. Vitamin C or a derivative thereof can be in any physical form, for example, a liquid, a semisolid, a solid, or a heat stable form.

The vitamin C content of the compositions of the invention is from 350 to 500 ppm.

The compositions of the invention are useful for a variety of human and non-human animals, including avian, bovine, canine, equine, feline, hicrine, murine, ovine, and porcine animals, and are particularly useful for companion animals such as canines and felines, including dogs and cats.

The invention provides a composition for use in promoting oral health in an animal, which composition comprises at least one antioxidant, wherein the at least one antioxidant comprises vitamin C in an amount of from 350 to 500 ppm and vitamin E in an amount of from 1000 to 1500 ppm, and wherein the use comprises causing the animal to ingest the composition. In one embodiment, the compositions promote oral health by reducing the accumulation of plaque. In others, the compositions promote oral health by promoting gingival and/or periodontal health even where plaque accumulation is not reduced. It is particularly surprising that the compositions, as shown in Example 1, keep gingivitis at a low baseline level even as plaque, normally a factor promoting gingivitis, accumulates. In a further embodiment, the compositions promote oral health by promoting gum and/or periodontal health.

Compositions suitable for ingestion by a companion animal include foods, supplements, treats, snacks, and toys (typically chewable and consumable toys).

In one embodiment, the composition comprising the one or more antioxidants can be fed to the animal as a component of its food. The food meets the animal's ordinary nutritional requirements, which a skilled artisan can determine based upon the animal's species, age, sex, weight, and other factors. For example, a typical diet for a canine of 1-6 years of age contains on a dry matter basis about 23% protein, about 15% fat, about 0.6% phosphorus, 0.6% calcium, and about 0.3% sodium. A typical diet for older canines and felines is shown in Table 1.

**Table 1**

| Typical Composition of Diet for Older Canines and Felines | | |
|---|---|---|
| Component | Canine | Feline |
| crude protein (% dry matter) | 15-25 | 26-50 |
| crude fat (% dry matter) | 7-20 | 10-30 |
| crude fiber (% dry matter) | >2 | <10 |
| calcium (% dry matter) | 0.5-1.2 | 0.6-1.5 |
| phosphorus (% dry matter) | 0.25-1.2 | 0.5-1.5 |
| sodium (% dry matter) | 0.15-0.5 | 0.15-0.5 |
| magnesium (% dry matter) | 0.05-0.2 | 0.05-0.15 |
| energy density ¹ | 3.0-4.5 | 3.5-5.0 |

| | | |
|---|---|---|
| ¹ kcal ME (metabolizable energy) per kg food (dry matter) | | |

In another embodiment, the composition is a food supplement comprising one or more antioxidants. Supplements include, for example, a feed or pet food used with another feed or pet food to improve the nutritive balance or performance of the total. Supplements include compositions that are fed undiluted as a supplement to other feeds or pet foods, offered free choice with other parts of an animal's ration that are separately available, or diluted and mixed with an animal's regular feed or pet food to produce a complete feed or pet food. AAFCO, for example, provides a discussion relating to supplements in the American Feed Control Officials, Inc. Official Publication, p. 220 (2003). Supplements can be in various forms including, for example, powders, liquids, syrups, pills, encapsulated compositions, *etc*.

In another embodiment, the composition is a treat comprising one or more antioxidants. Treats include, for example, compositions that are given to an animal to entice the animal to eat during a non-meal time. Treats for canines include, for example, dog biscuits in the shape of dog bones. Treats can be nutritional, wherein the composition comprises one or more nutrients, and can, for example, have a composition as described above for food. Non-nutritional treats encompass any other treats that are non-toxic. The one or more antioxidants, for example, can be coated onto the treat, incorporated into the treat, or both.

In a further embodiment, the composition is a toy comprising one or more antioxidants. Toys include, for example, chewable toys. Toys for dogs include, for example, artificial bones. The one or more antioxidants, for example, can be present in a coating on the surface of the toy or on the surface of a component of the toy, or can be incorporated partially or fully throughout the toy, or both. In an embodiment, the one or more antioxidants are orally accessible by the intended user. Illustrative toys suitable for modification in accordance with the invention are disclosed in U.S. Patent No. 5,339,771, U.S. Patent No. 5,419,283, and references disclosed therein. The invention provides both partially consumable toys (*e.g.*, toys comprising plastic components) and fully consumable toys (*e.g.*, rawhides and various artificial bones). The invention also provides toys for both human and non-human use, particularly for companion, farm, and zoo animal use, and particularly for dog or cat use. The terms "treat" and "toy" can be considered interchangeable. However, in general a treat is fully edible and a toy has an edible coating.

In preparing a composition of the present invention, the one or more antioxidants can be incorporated into the composition during formulation processing, such as during and/or after mixing of other components of the composition. Distribution of these components into the composition can be accomplished by any conventional method including standard mixing procedures known to skilled artisans.

Compositions of the present invention (particularly foods) can be prepared in a canned or wet form using conventional pet food processes. In one embodiment, ground animal (*e.g.*, animal, poultry, and/or fish) proteinaceous tissues are mixed with other ingredients, including for example animal fats and vegetable oils, cereal grains, other nutritionally balancing ingredients, special purpose additives (*e.g.*, vitamin and mineral mixtures, inorganic salts, cellulose and beet pulp, bulking agents, and the like); and water sufficient for processing is also added.

Compositions of the present invention (particularly foods) can be prepared in a dry form using conventional processes. In one embodiment, dry ingredients, including, for example, animal protein sources, plant protein sources, grains, *etc*., are ground and mixed together. Moist or liquid ingredients, including fats, oils, animal protein sources, water, etc., are then added to and mixed with the dry mix. The mixture is then processed into kibbles or similar dry pieces. Kibble is often formed using an extrusion process in which the mixture of dry and wet ingredients is subjected to mechanical work at a high pressure and temperature, and forced through small openings and cut off into kibble by a rotating knife. The wet kibble is then dried and optionally coated with one or more topical coatings that can include, for example, flavors, fats, oils, powders, and the like. Kibble also can be made from the dough using a baking process, rather than extrusion, wherein the dough is placed into a mold before dry-heat processing. Kibble also can be made from a food matrix undergoing pelletization. It is important to note that the antioxidants can be incorporated into the food composition by adding the antioxidants, for example, to the above-described mixtures before extrusion or by coating the extruded kibble or pellets with, for example, at least one antioxidants as an ingredient of a topical coating.

Treats of the present invention can be prepared by, for example, an extrusion or baking process similar to those described above for dry food. Other processes also can be used to either apply a coating comprising the one or more antioxidants on the exterior of existing treat forms, or inject the one or more antioxidants into an existing treat form. Animal toys of the present invention are typically prepared by coating any existing toy with a composition comprising at least one antioxidant.

The antioxidant or mixture of antioxidants can be fed to an animal as a component of its food or as a food supplement. The quantities provided in the food, all on a dry matter basis, are stated herein as the active material. The antioxidant amount should not exceed a maximum above which toxicity is brought about. Preferably, the antioxidant, or mixture thereof, is fed to the animal in a total amount effective to promote oral health. What constitutes an effective amount varies depending on the species of the animal, the type of antioxidant(s) and other factors. One of skill in the art will, by routine testing based on the disclosure herein; readily establish a total antioxidant amount having oral health promoting effects in any particular situation.

Also described are kits suitable for administering one or more antioxidants to an animal. The kits comprise in separate containers in a single package or in separate containers in a virtual package, as appropriate, at least one antioxidant and at least one of (1) one or more ingredients suitable for consumption by an animal, (2) instructions for how to combine the antioxidants and other kit components to produce a composition useful for promoting oral health, and (3) instructions for how to use the antioxidants and other components of the present invention, particularly to promote oral health. When the kit comprises a virtual package, the kit is limited to instructions in a virtual environment in combination with one or more physical kit components. The kit contains the antioxidants and other components in amounts sufficient to promote oral health. Typically, the antioxidants and the other suitable kit components are admixed just prior to consumption by an animal. In one embodiment, the kit contains a packet containing one or more one or more antioxidants and a container of food for consumption by an animal. The kit may contain additional items such as a device for mixing the antioxidants and ingredients or a device for containing the admixture, e.g., a food bowl. In another embodiment, the antioxidants are mixed with additional nutritional supplements such as vitamins and minerals that promote good health in an animal.

Also described is a means for communicating information about or instructions for one or more of (1) using one or more antioxidants to promote oral health, (2) admixing one or more antioxidants with the other components of the present invention, (3) administering one or more antioxidants to an animal, alone or in combination with the other elements of the present invention, and (4) using the kits of the present invention for promoting oral health. The means comprises a document, digital storage media, optical storage media, audio presentation, or visual display containing the information or instructions. In certain embodiments, the communicating means comprises a document, digital storage media, optical storage media, audio presentation, or visual display containing the information or instructions. Preferably, the communication means is a displayed web site or a brochure, product label, package insert, advertisement, or visual display containing such information or instructions. Useful information includes one or more of (1) methods and techniques for combining and administering the antioxidants and/or other components and (2) contact information for animals or their caregivers to use if they have a question about the invention and its use. Useful instructions include amounts for mixing and administration amounts and frequency. The communication means is useful for instructing on the benefits of using the present invention and communicating the approved methods for administering the invention to an animal.

In another, the invention provides for the use of the composition to prepare a medicament for promoting oral health. Generally, medicaments are prepared by admixing a compound or composition with excipients, buffers, binders, plasticizers, colorants, diluents, compressing agents, lubricants, flavorants, moistening agents, and other ingredients known to skilled artisans to be useful for producing medicaments and formulating medicaments that are suitable for administration to an animal.

### EXAMPLES

The invention can be further illustrated by the following examples of preferred embodiments thereof, although it will be understood that these examples are included merely for purposes of illustration and are not intended to limit the scope of the invention unless otherwise specifically indicated.

### Example 1

This example illustrates that gingival inflammation does not increase in cats fed a nutritionally complete feline pet food supplemented with vitamins E and C in various combinations.

Thirty-eight cats were randomly assigned to one of the four groups A-D. A nutritionally complete feline pet food was supplemented with 4 different levels of the antioxidants vitamins C and vitamin E. The groups A-D are designated as follows:

| | |
|---|---|
| A | vitamin C - 35 ppm, vitamin E - 550 ppm |
| B | vitamin C - 35 ppm, vitamin E - 1000 ppm |
| C | vitamin C - 350 ppm, vitamin E - 550 ppm |
| D | vitamin C - 350 ppm, vitamin E - 1000 ppm |

Groups A-C are comparative examples and Group D is according to the present invention.

Prior to study initiation, all cats received a complete dental cleaning including supragingival and subgingival plaque and tartar removal and polishing of all exposed tooth surfaces with rubber cup and pumice. Each cat received daily tooth brushing with a soft tooth brush and a pet dentifrice for a period of 5 days. Tooth brushing was discontinued 48 hours prior to baseline evaluations. At Day 0, all cats were evaluated for baseline plaque accumulation and gingival inflammation. Each cat was given a complete dental cleaning and four weeks later cats were again evaluated for plaque accumulation and gingival inflammation.

In a previous study using a similar model, cats were fed a typical dry grocery cat food having no vitamin C and 65 ppm vitamin E. This group of cats showed 142% increase in plaque and 52.4% increase in gingivitis over a 4 week period.

As shown in Table 2 below, there was no substantial change in the level of gingivitis from baseline over the four week period in groups A-D. This is in spite of the fact that each of groups A-D exhibited an increase from baseline values in level of plaque

**Table 2**

| Change Over Baseline in Plaque and Gingivitis Scores | | |
|---|---|---|
| Group | % change | |
| | plaque | gingivitis |
| A | 30.9 | -6.5 |
| B | 24.6 | 6.9 |
| C | 58.0 | -3.8 |
| D | 64.4 | -14.8 |

These data demonstrate that a feline pet food supplemented with various combinations of vitamins E and C can provide a protective effect, as indicated by no substantial change in level of inflammation, to gingival tissues over a four week period.

## Claims

1. A composition for use in promoting oral health in an animal, which composition comprises at least one antioxidant, wherein the at least one antioxidant comprises vitamin C in an amount of from 350 to 500 ppm and vitamin E in an amount of from 1000 to 1500 ppm, and wherein the use comprises causing the animal to ingest the composition.

2. The composition of claim 1, for the use according to claim 1, which further comprises at least one antioxidant selected from the group consisting of vitamin A, lipoic acid, astaxanthin, beta-carotene, L-carnitine, coenzyme Q10, glutathione, lycopene, lutein, N-acetylcysteine, soy isoflavones, S-adenosylmethionine, taurine, tocotrienols, spinach, tomato, citrus fruit, grape, carrot, broccoli, green tea, ginkgo biloba, corn gluten meal, rice bran, algae, curcumin, marine oil, fruits, vegetables, yeast, carotenoids, flavonoids, polyphenols, and mixtures thereof.

3. The composition of claim 1 or claim 2 for the use according to claim 1 or claim 2, wherein the animal is a companion animal.

4. The composition of any preceding claim for the use of any preceding claim, wherein the animal is a canine.

5. The composition of any of claims 1 to 3 for the use of any of claims 1 to 3, wherein the animal is a feline.

6. The composition of any preceding claim for the use of any preceding claim, wherein the composition is a food.

7. The composition of any of claims 1 to 5 for the use of any of claims 1 to 5, wherein the composition is a treat or a toy.

8. The composition of any of claims 1 to 5 for the use of any of claims 1 to 5, wherein the composition is a supplement.

9. The composition of any preceding claim for the use of any preceding claim, wherein the use comprises the mitigation, prevention, or treatment of gingival or periodontal health.

10. The composition of claim 9 for the use according to claim 9, wherein the use comprises the mitigation, prevention, or treatment of gingivitis.

## Patentansprüche

1. Eine Zusammensetzung, die zur Förderung der Mundgesundheit eines Tiers verwendet wird und mindestens ein Antioxidationsmittel umfasst, wobei das zumindest eine Antioxidationsmittel Vitamin C in einer Menge von 350 bis 500 ppm sowie Vitamin E in einer Menge von 1000 bis 1500 ppm enthält, und wobei das Tier bei der Verwendung zur Aufnahme der Zusammensetzung veranlasst wird.

2. Die Anspruch 1 entsprechende Zusammensetzung zur Anspruch 1 entsprechenden Verwendung, die ferner mindestens ein Antioxidationsmittel umfasst, das aus der Gruppe ausgewählt ist, die aus Vitamin A, Liponsäure, Astaxanthin, Beta-Carotin, L-Carnitin, Coenzym Q10, Glutathion, Lycopin, Lutein, N-Acetylcystein, Soja-Isoflavonen, S-Adenosylmethionin, Taurin, Tocotrienolen, Spinat, Tomaten, Zitrusfrüchten, Trauben, Möhren, Broccoli, Grüntee, Ginkgo Biloba, Maiskleber, Reiskleie, Algen, Curcumin, Marineöl, Früchten, Gemüse, Hefe, Carotinoiden, Flavonoiden, Polyphenolen und deren Mischungen besteht.

3. Die Anspruch 1 oder Anspruch 2 entsprechende Zusammensetzung zur Anspruch 1 oder Anspruch 2 entsprechenden Verwendung, wobei das Tier ein Haustier ist.

4. Die einem vorstehenden Anspruch entsprechende Zusammensetzung zu einer einem vorstehenden Anspruch entsprechenden Verwendung, wobei das Tier ein Hund ist. EP 05 855 196

5. Die einem der Ansprüche 1 bis 3 entsprechende Zusammensetzung zur Ansprüchen 1 bis 3 entsprechenden Verwendung, wobei das Tier eine Katze ist.

6. Die einem vorstehenden Anspruch entsprechende Zusammensetzung zu einer einem vorstehenden Anspruch entsprechenden Verwendung, wobei die Zusammensetzung ein Futter ist.

7. Die einem der Ansprüche 1 bis 5 entsprechende Zusammensetzung zu einer Ansprüchen 1 bis 5 entsprechenden Verwendung, wobei die Zusammensetzung ein Leckerbissen oder Spielzeug ist.

8. Die einem der Ansprüche 1 bis 5 entsprechende Zusammensetzung zur Ansprüchen 1 bis 5 entsprechenden Verwendung, wobei die Zusammensetzung eine Nahrungsergänzung ist.

9. Die einem vorstehenden Anspruch entsprechende Zusammensetzung zu einer einem vorstehenden Anspruch entsprechenden Verwendung, die die Linderung, Verhütung oder Behandlung von gingivaler oder parodontaler Gesundheit umfasst.

10. Die einem vorstehenden Anspruch entsprechende Zusammensetzung zu einer einem vorstehenden Anspruch entsprechenden Verwendung, die die Linderung, Verhütung oder Behandlung von Gingivitis umfasst.

## Revendications

1. Une composition destinée à être utilisée pour l'amélioration de la santé bucco-dentaire chez un animal, laquelle composition comprend au moins un antioxydant, dans laquelle le au moins un antioxydant comprend de la vitamine C en raison de 350 à 500 ppm et de la vitamine E en raison de 1000 à 1500 ppm, et dans laquelle l'utilisation comprend de faire ingérer la composition par l'animal.

2. La composition de la revendication 1, pour l'utilisation selon la revendication 1, qui comprend en outre au moins un antioxydant sélectionné du groupe consistant de vitamine A, acide lipoique, astaxanthine, bêta-carotène, L-carnitine, coenzyme Q10, glutathion, lycopène, lutéine, N-acétylcystéine, isoflavones de soja, S-adénosylméthionine, taurine, tocotriénols, épinard, tomate, agrumes, raisin, carotte, brocoli, thé vert, gingko biloba, farine de gluten de maïs, son de riz, algues, curcumine, huile de poisson, fruits, légumes, levure, caroténoïdes, flavonoïdes, polyphénols et de mélanges de ceux-ci.

3. La composition de la revendication 1 ou de la revendication 2 pour l'utilisation selon la revendication 1 ou la revendication 2, dans laquelle l'animal est un animal de compagnie.

4. La composition de l'une quelconque des revendications précédentes pour l'utilisation selon l'une quelconque des revendications précédentes, dans laquelle l'animal est un chien.

5. La composition de l'une quelconque des revendications de 1 à 3 pour l'utilisation selon l'une quelconque des revendications de 1 à 3, dans laquelle l'animal est un chat.

6. La composition de l'une quelconque des revendications précédentes pour l'utilisation selon l'une quelconque des revendications précédentes, dans laquelle la composition est une composition alimentaire.

7. La composition de l'une quelconque des revendications de 1 à 5 pour l'utilisation selon l'une quelconque des revendications de 1 à 5, dans laquelle la composition est une friandise ou un jouet.

8. La composition de l'une quelconque des revendications de 1 à 5 pour l'utilisation selon l'une quelconque des revendications de 1 à 5, dans laquelle la composition est un supplément.

9. La composition de l'une quelconque des revendications précédentes pour l'utilisation selon l'une quelconque des revendications précédentes, dans laquelle l'utilisation comprend l'atténuation, la prévention, ou le traitement de problèmes de santé gingivale ou parodontale.

10. La composition de la revendication 9 pour l'utilisation selon la revendication 9, dans laquelle l'utilisation comprend l'atténuation, la prévention ou le traitement de la gingivite.
